## Europäisches Patentamt
## European Patent Office
## Office européen des brevets

(11) Veröffentlichungsnummer: **0 105 194**
**B1**

(12) **EUROPÄISCHE PATENTSCHRIFT**

(45) Veröffentlichungstag der Patentschrift: **25.05.88**

(51) Int. Cl.⁴: **G 01 N 33/50**, A 61 B 10/00, B 04 B 5/04

(21) Anmeldenummer: **83108568.3**

(22) Anmeldetag: **31.08.83**

(54) **Zentrifugationskammern zur zytodiagnostischen Präparation von Epithelzellen und deren Verwendung.**

(30) Priorität: **02.09.82 DE 3232581**

(43) Veröffentlichungstag der Anmeldung:
**11.04.84 Patentblatt 84/15**

(45) Bekanntmachung des Hinweises auf die Patenterteilung:
**25.05.88 Patentblatt 88/21**

(84) Benannte Vertragsstaaten:
**AT BE CH DE FR GB IT LI LU NL SE**

(56) Entgegenhaltungen:
EP-A-0 045 368
EP-A-0 045 369
DE-A-2 823 490
DE-A-3 041 131
THE JOURNAL OF HISTOCHEMISTRY AND CYTOCHEMISTRY, Band 27, no. 1, 1979, K. OTTO, H. HÖFFKEN, H.J. SOOST
"Sedimentation velocity separation: A preparation method for cervical samples", S. 14-18

(73) Patentinhaber: **Firma Andreas Hettich
Gartenstrasse 100
D-7200 Tuttlingen (DE)**

(72) Erfinder: **Eberle, Günter
Gartenstrasse 100
D-7200 Tuttlingen (DE)**

(74) Vertreter: **Riebling, Peter, Dr.-Ing.
Patentanwälte Dr.-Ing., Dipl.-Ing., Ing.(grad.)
Günter Riebling Dr.-Ing., Dipl.-Ing. Peter Riebling
Rennerle 10 Postfach 3160
D-8990 Lindau (DE)**

(56) Entgegenhaltungen:
ACTA HISTOCHEMICA, suppl.-Band XXI, 1980, K. OTTO "Zelldeposition für automatisiertes Prescreening gynäkologischer Präparate"
Biotechnische Umschau Band 2, 1978, Heft 4 K. Otto et al. "Ein Verfahren zur isolierten Deposition von Zellen der Cervix uteri auf Glasobjektträgern" Seiten 128 bis 131
Tumor Diagnostik Band 2, 1981 H.-J. Soost et al. "Materialgewinnung und Präparation für ein automatisches Präscreening des Zervixkarzinoms" Seiten 35 bis 38

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Die Erfindung betrifft Zentrifugationskammern zur zytodiagnostische Präparation von in einer Suspensionsprobe enthaltenen Epithelzellen nach den Ansprüchen 1 und 4 sowie deren Verwendung nach dem Anspruch 7.

Es geht darum, die auf einem Abstrich erhaltenen Epithelzellen zu vereinzeln und sie auf einen Objektträger möglichst gleichmäßig nebeneinander zu deponieren. Der Schmutz sowie die zahlreichen Zellkerne, Erythrozyten, Leukozyten uzw. werden durch Fraktionierung weitgehend abgetrennt und als Überstand abpipettiert (leichte Fraktion). Beide Fraktionen, die distale und die proximale Fraktion (Überstand), werden dann separat auf je einen Objettträger aufzentrifugiert, der eine Objektträger dient zum Erkennen, Auswerten und Zählen der Epithelzellen, der andere Objektträger dient zum Nachweis, daß nicht wesentliche Teile von gesuchten Epithelzellen noch im Überstand vorhanden sind, und daß der erste Objektträger ein repräsentatives Bild des Abstrichs darstellt.

Das bisherige Verfahren ist in der Zeitschrift Biotechnische Umschau, 1978, Heft 4, Seiten 128—131, unter der Überschrift "Ein Verfahren zur isolierten Deposition von Zellen der Cervix uteri auf Glasobjektträger" und in der Zeitschrift "Tumor Diagnostik 2, 1981, Seiten 35—38" und in der Zeitschrift "The Journal of Histochemistry and Cytochemistry, vol. 27. 1979, Seiten 14—18" sowie in der Zeitschrift "Acta histochemica, Supp.-Band XXI, 1980, Seiten 137—145" beschrieben.

Bei den bekannten Verfahren mußte also insgesamt drei Mal zentrifugiert werden. Im ersten Arbeitsgang wird nach dem Aufschütteln der Suspensionsproben mit einem Watteträger, wobei der Watteträger die zu untersuchenden Zellen enthält, der Watteträger und grobe Verunreinigungen dadurch entfernt, daß die Suspension durch ein Nylonsieb gegossen wird.

Die Suspensionsproben werden dann im zweiten Arbeitsgang mit einer Zentrifuge für die Dauer von 10 Minuten bis etwa 2000 UpM absedimentiert. Die Suspensionsprobe wird dann im dritten Arbeitsgang aus dem Probenröhrchen abgesaugt und auf ein Trennmedium (Plasmasteril) überschichtet. Es erfolgt im vierten Arbeitsgang ein erneuter Zentrifugationsvorgang von etwa 10 Minuten bei 500 UpM. Die so erhaltene fraktionierte Säule wird im fünften Arbeitsgang dadurch präpariert, daß die proximale Fraktion (Überstand) und die distale Fraktion (Plasma) durch Abpipettierung getrennt werden.

Hiernach wird dann im sechsten Arbeitsgang ein Objektträger in eine Zentrifugationskammer eingelegt, wobei in die eine Bohrung die proximale Fraktion (Überstand) und in die andere Bohrung die distale Fraktion (Plasma) eingegeben wird.

Es erfolgt dann mit dem siebten Arbeitsgang der dritte Zentrifugiervorgang, wobei etwa 10 Minuten bei 2000 UpM zentrifugiert wird.

Während des Zentrifugiervorgangs wandern die schwereren Epithelzellen in der Flüssigkeit aufgrund der auf sie ausgeübten Zentrifugalkraft in Richtung zum Objektträger und lagern sich in Form eines Sedimentes an.

Nach der Aufsedimentation der zu untersuchenden Zellen auf den Objektträger wird der flüssige Rückstand in der Zentrifugationskammer mit eine Pumpe abgesaugt und die mit den zu untersuchenden Zellen beschichteten Objektträger in eine 96%-ige Alkohollösung gestellt. Es erfolgt dann die Pap-Färbung des Objektträgers. Auf dem einen Feld des Objektträgers, dessen Bohrung mit der distalen Fraktion gefüllt wurde, werden die Epithelzellen aufsedimentiert, während auf dem benachbarten Feld des gleichen Objektträgers, dem die Bohrung zugeordnet ist, die mit der proximalen Fraktion (Überstand) gefüllt ist, eine andere Art von Zellen aufsedimentiert wird. Dieses aufsedimentierte Feld dient nur dem Nachweis, daß nicht wesentliche Teile von gesuchten Epithelzellen noch im Überstand vorhanden sind, daß also der erste Objettträger ein repräsentatives Bild des Abstrichs darstellt.

Nachteil bei dem bekannten Verfahren ist, daß ein dreimaliger, recht arbeits- und zeitaufwendiger, Zentrifugationsvorgang vorgenommen werden muß. Besonders störend ist der zweite Zentrifugationsvorgang, bei dem beispielsweise 4 ml der Stammlösung auf 6 ml des Trennmediums überschichtet werden, und die so hergestellte Untersuchungsflüssigkeit duch Abzentrifugieren in eine proximale Fraktion (Überstand) und eine distale Fraktion (Plasma) aufgetrennt wird, wobei dann in zeitaufwendiger Weise beide Fraktionen durch Abpipettierung getrennt werden müssen und in getrennte Bohrungen der Zentrifugationskammer eingefüllt werden müssen.

Die vorliegende Erfindung hat sich die Aufgabe gestellt, das bekannte Verfahren weniger zeit- und arbeitsaufwendig zu gestalten, so daß ein oder mehrere Arbeitsschritte zusammengefaßt in einem einzigen Arbeitsvorgang ausgeführt werden können.

Mit der Hinzufügung einer dritten Bohrung in Serie zu der zweiten Bohrung wird der Vorteil erreicht, daß nun auch eine Färbung oder ein anderweitige Behandlung der auf den Objektträger aufsedimentierten Zellkörper möglich ist. Die dritte Bohrung wirkt hierbei als Abfallkammer für die aus der ersten und zweiten Bohrung in die dritte Bohrung abgeleitete Flüssigkeit. Nach erfolgter Absedimentation der Epithelzellen auf dem Objektträgerfeld, das jeweils unter der ersten und zweiten Bohrung angeordnet ist, werden beiden Bohrungen entleert. Hierzu wird der Inhalt der ersten Bohrung durch Beaufschlagung mit Preßluft in die zweite Bohrung übergeführt und füllt diese Bohrung auf. Die Preßluft wirkt über die erste Bohrung und über den Verbindungskanal zwischen der ersten und der zweiten Bohrung auf die zweite Bohrung und verdrängt dort die dort angesammelte Flüssigkeit über einen weiteren Kanal in die dritte, benachbarte Bohrung. In dieser Bohrung sammelt sich nun der Inhalt der ersten und zweiten Bohrung an. Nachdem die erste und zweite Bohrung vollständig durch Beaufschlagung mit Preßluft entleert wurden, wird Färbeflüssigkeit in die erste Bohrung eingeführt.

Es ist auf einfache Weise möglich, neben der Durchführung des Zentrifiervorganges zur Absedimentation von Zellkörpern auf verschiedenen Flächen des Objektträgerfeldes auch noch gleichzeitig einen Färbevorgang durchzuführen, ohne daß hierbei der Objektträger aus der Zentrifugationskammer gelöst werden muß und umständlichen Färbeverfahren unterworfen werden muß.

Nach erfolgter Entfärbung der Färbeflüssigkeit aus der ersten und zweiten Bohrung ist es dann möglich, noch weitere Flüssigkeiten einzubringen und die Objektträgerfelder der ersten und zweiten Bohrung entsprechend damit zu beaufschlagen. Dies ist beispielsweise mit Einbringung einer Fixierflüssigkeit möglich, welche die angefärbten Zellen auf dem Objektfeld fixiert. Ebenso ist die Einbringung spezieller Spülflüssigkeiten möglich, wobei immer jeweils der beschriebene Arbeitsvorgang durchgeführt wird.

Durch Anordnung weiterer Reservoirs, die andere Behandlungsflüssigkeiten enthalten, und die in Flüssigkeitsverbindung mit der ersten Bohrung der Zentrifugationskammer stehen, ist es möglich, während des Zentrifugationsvorgangs beliebig viele Behandlungsflüssigkeiten in die erste und zweite Bohrung einzubringen und schließlich auch wieder auszutragen.

In zusammengefaßter Form werden nachfolgend die verschiedenen Austreibe-Möglichkeiten erläutert, wie es gelingt, Behandlungsflüssigkeiten von der ersten Bohrung in eine zweite zu bringen und ggf. die zweite Bohrung zu entleeren und die dortige Behandlungsflüssigkeit in eine dritte und von dort aus in weitere Bohrungen zu bringen.

Die Fließverbindung besteht aus einer im Querschnitt veränderbaren Überlaufbohrung, wobei die eine Bohrung in eine Bohrung größeren Durchmessers und eine mit dieser fluchtenden Bohrung kleineren Durchmessers aufgeteilt ist und ferner die Überlaufbohrung vom Ansatz der größeren in die kleinere Bohrung ausgeht.

Die Kammer mit der abgesetzten Bohrung ist die Einfüllkammer für die Probensuspension und die Kammer mit der durchgehenden Bohrung stellt die Überlaufkammer dar. Die Höhe der abgesetzten Bohrung bildet etwa die Trennschicht der beiden ungleich schweren Fraktionen.

Eine Klemmschraube dient zum Einregulieren der Überlauföffnung, d. h., zur Veränderung des Querschnittes der Überlaufbohrung Mit Beginn der Zentrifugation, d.h., Ausschwingen des Gefäßträgers (Schaukel) beginnt bereits das Überströmen in der zentrifugalkraft-entgegengesetzten Richtung. Ein winziger Spalt an der Klemmschraube läßt genügend Überstand (proximale Fraktion) in die zweite Bohrung überströmen; die Kraft wird hierbei durch die Zentrifugalkraft erzeugt.

Es ist eine Überlaufkammer mit Verschlußstück vorgesehen. Dieses Verschlußstück weist am Boden eine Kerbe auf. Die Abmessung dieser Kerbe definiert den Durchlaßquerschnitt von einer Bohrung in die andere. Die Kerbe wird unter Zentrifugalkraft auf den Durchlaßquerschnitt gepreßt. Man kann Verschlußstücke mit unterschiedlich tiefen Kerben verwenden, um für jeden Fall der Suspensionslösung einen bestimmten Durchgangsquerschnitt von einer Bohrung in die andere zu erreichen.

Wesentlich bei dem erstgenannten Beispiel mit Klemmschraube und bei dem zweitgenannten Beispiel mit Verschlußstück ist, daß die Probenkammern für ausschwingende Gehänge konzipiert sind, d.h., sie stehen im Ruhezustand senkrecht und im Betriebszustand waagerecht. Der Objektträger liegt mit seiner zu beschichtenden Ebene im Ruhezustand waagerecht und im Betriebszustand senkrecht.

Das Überströmen durch die Fließverbindung von der einen Bohrung in die andere erfolgt durch Druckluft. Die hierfür vorgesehenen Probenkammern werden für eine Zentrifuge mit Topfrotor verwendet, bei der die Zentrifugationskammer nicht um 90° sich während des Zentrifugationsvorganges verlagert. Die Probenkammern werden in den Topfrotor eingesetzt, wobei die Ausnehmungen für den Luftanschluß auf die Dichtungsstücke für den Luftauslaß zu liegen kommen. Die Zentrifugationskammern werden dann über einen senkrechten Einfüllstutzen gefüllt und zwar, wie bei den übrigen Beispielen auch, zunächst mit sechs ml Plasmasteril und mit vier ml Zellsuspension hierüber geschichtet. Ein Deckel mit elastischer Platte, z.B. eine Gummiplatte, verschließt alle Kammern gemeinsam, so daß ein geschlossenes System entsteht. Beim Lauf der Zentrifuge werden durch die Schwerkraft die beiden Fraktionen umgeschichtet.

Die Trennfläche, die in Ruhe waagerecht war, orientiert sich während des Zentrifugationsvorganges senkrecht. Der Objektträger bleibt hierbei in senkrechter Position. Bei geringer Zentrifugalkraft (ca. 60 g) dringen die Epithelzellen in das Plasmasteril ein. Der Überstand, der ansonstem gem. dem ersten Ausführungsbeispiel (Überlaufbohrung, Verschlußstück) ziemlich lange brauchen würde zum Überfließen in die andere Bohrung, wird mittels Luftdruck durch die Zentrifugenwelle über den hohlen Boden des Topfrotors und über die Anschlußstücke in den Einfüllstutzen eingeführt, so daß das Überströmen durch die Überlaufbohrung beschleunigt erfolgt.

Bei diesen mit Druckluft beaufschlagten Probenkammern gibt es zwei verschiedene Ausführungen. Die eine weist eine Probenkammer mit abgesetzter Bohrung auf, wobei diese Bohrung das Plasmasteril und die Probensuspension aufnimmt. Die Überlaufbohrung mündet hierbei am Ansatz des Übergangs der größeren Bohrung in die kleinere Bohrung.

Die zweite Lösung bezieht sich auf eine Probenkammer mit durchgehender Bohrung, in welche das Plasmasteril und die Probensuspension eingefüllt wird. Es wird ein Verbindungskanal vorgeschlagen, der parallel und in geringem Abstand zu dieser Bohrung im Material der Probenkammer eingearbeitet ist, wobei dieser Verbindungskanal zunächst keinerlei Verbindung zu dieser Bohrung aufweist, und mit seiner Stirnseite auf dem Objektträger mündet.

Das besondere an diesem separaten Verbindungskanal ist, daß man in jedem beliebigen Abstand vom Objektträger entfernt mit einer Nadel ein Verbindugsloch in die Wandung zwischen der die Probensuspension aufnehmenden Bohrung und dem Verbindungskanal einstechen kann, und daß dadurch die Restflüssigkeit in der die Probensuspension aufnehmenden Bohrung variierbar ist.

Eine solche Probenkammer kann beispielsweise aus einem gespritzten Kunststoffmaterial bestehen, so daß es ohne weiteres möglich ist, mit einer Nadel die Wandung der die Probelflüssigkeit aufnehmenden Bohrung anzustechen, um eine Verbindung zum Verbindungskanal zu schaffen. Nach der Erfindung werden also drei verschieden Möglichkeiten zur Steuerung des Flüssigkeitsflusses von einer Bohrung zu der benachbarten Bohrung vorgesehen.

Es handelt sich hierbei entweder um eine Klemmschraube, oder ein Verschlußstück, welche den Durchlaßquerschnitt des Verbindungskanals verändern oder um die Einleitung von Druckluft, welche die Strömungsgeschwindigkeit im Kanal erhöht, wobei dann eine Klemmschraube oder ein Verschlußstück entfallen kann.

Im folgenden wird die Erfindung anhand von mehrere ausführungswege darstellenden Zeichnungen näher erläutert.

Es zeigen:

Figur 1: Schnitt durch eine zentrifugationskammer in einer ersten Ausführungsform gemäß der Linie I—I in Fig. 2, wobei die Flüssigkeitsverteilung während des Zentrifugationsvorgangs dargestellt ist;

Figur 2: Stirnansicht der Zentrifugationskammer in Richtung des Pfeiles II in Fig. 3;

Figur 3: Draufsicht auf die Zentrifugationskammer in Richtung des Pfeiles III in Figur 2;

Figur 4: schematisiert dargestellt Ansicht des Aufbaus und der Fraktionierung der Trennsäule in einem Zentrifugenröhrchen, vor (links) und nach (rechts) Sedimentation der Probe;

Figur 5: Schnitt gemäss der Linie V—V in Figur 6 durch eine zweite Ausführungsform einer Zentrifugationskammer, wobei die Flüssigkeitsverteilung während des Zentrifugationsvorgangs dargestellt ist;

Figur 6: Stirnansicht der Zentrifugationskammer in Richtung des Pfeils VI in Figur 7;

Figur 7: Draufsicht auf die Zentrifugationskammer des zweiten Ausführungsbeispiels gemäss dem Pfeil VII in Figur 6;

Figur 8: Bodenansicht des Verschlußstückes;

Figur 9: Seitenansicht des Verschlußstückes nach Figur 8;

Figur 10: Seitenansicht einer Schaukel (Schaukelgehänge) zur Verwendung mit den Zentrifugwtionskammer nach dem Figuren 1 bis 3 und 5 bis 7 in der Seitenansicht;

Figur 11: Draufsicht auf die Schaukel in Richtung des Pfeiles XI in Figur 10;

Figur 12: Seiten-Teilansicht der Schaukel;

Figur 13: Schnitt durch ein drittes Ausführungsbeispiel einer Zentrifugationskammer; gemäss Linie XIII—XIII in Figur 15; wobei die Flüssigkeitsverteilung während des Zentrifugationsvorgangs dargestellt ist;

Figur 14: Stirnansicht der Zentrifugationskammer nach Figur 13;

Figur 15: Draufsicht auf die Zentrifugationskammer in Richtung des Pfeiles XV in Figur 13;

Figur 16: Schnitt entlang der Linie XVI—XVI in Figur 18 durch ein viertes Ausführungsbeispiel einer Zentrifugationskammer; wobei die sich während des Zentrifugationsvorgangs ergebende Flüssigkeitsverteilung dargestellt ist;

Figur 17: Stirnansicht der Zentrifugationskammer nach Figur 16;

Figur 18: Draufsicht auf die Zentrifugationskammer in Richtung des Pfeiles XVIII in Figur 16;

Figur 19: schematisiert gezeichneter Schnitt durch eine Topfrotor mit Beispiel des Einsatzes zweier verschiedener Zentrifugationskammern;

Figur 20: Draufsicht auf den Topfrotor nach Figur 19 mit Darstellung des Einsatzes einer Zentrifugationskammer nach dem Ausführungsbeispiel der Figuren 13 bis 15;

Figur 21: Schnitt durch ein viertes Ausführungsbeispiel einer Zentrifugationskammer gemäss der Linie XXI—XXI in Figur 23;

Figur 22: Stirnansicht der Zentrifugationskammer nach Figur 21;

Figur 23: Draufsicht auf die Zentrifugationskammer nach Figur 21 in Richtung des Pfeiles 23 in Figur 21;

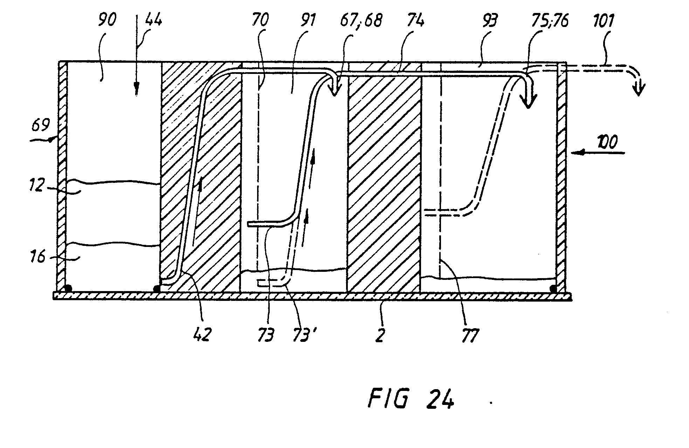

Figur 24: schematisiert gezeichneter Längsschnitt durch ein fünftes Ausführungsbeispiel einer Zentrifugationskammer;

Figur 25: schematisiert gezeichneter Längsschnitt durch ein sechstes Ausführungsbeispiel einer Zentrifugationskammer.

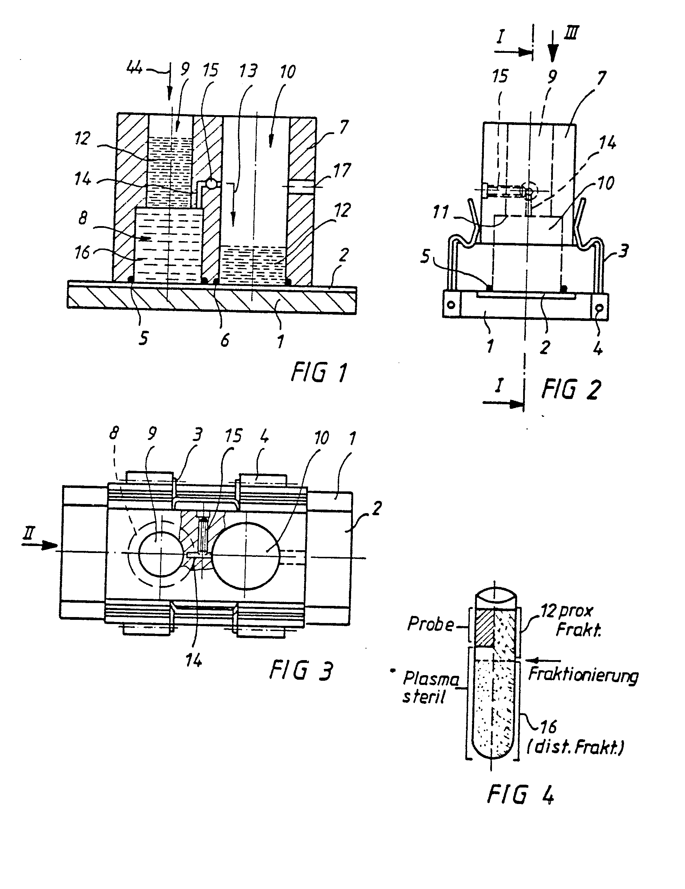

Anhand der Figur 4 soll zunächst die Herstellung der proximalen Fraktion 12 und der distalen Fraktion 16 schematisiert anhand eines Laborglases beschrieben werden. Die Probensuspension (in Figur 4 mit "Probe" bezeichnet), wirk auf ein Trennmedium (Plasma-Steril) aufgeschichtet. Es wird dann der Zentrifugiervorgang durchgeführt und die schwereren Epithelzellen in der Probensuspension wandern durch das Trennmedium in Richtung zum distalen Ende des Reagenzröhrchens. Nach erfolgtem Zentrifugiervorgang liegt dann eine proximale Fraktion 12 (Überstand) und eine distale Fraktion 16 (Plasma) vor. In der distalen Fraktion befinden sich bevorzugt und in hochkonzentrierter Form die zu untersuchenden Epithelzellen.

Bisher wurden in Höhe des Pfeiles "Fraktionierung" die proximale Fraktion 12 durch Abpipettierung von der distalen Fraktion 16 getrennt. Um danach dann die distale Fraktion eneut zu zentrifugieren, um die sort suspendierten Epithelzellen unter Einfluß der Zentrifugalkraft zu veranlussen, zum distalen Ende der Probenkammer (Bohrung zu wandern und sich auf einem dort angeordneten Objetträger anzulegen.

Anhand der Figuren 1—3 wird nun beschrieben, wie während des Zentrifugiervorgangs (also ohne Abpipettierung, wie üblich) die proximale Fraktion 12 von der distalen Fraktion 16 getrennt wird. Damit wird die eingangs beschriebene Abpipettierung zur Trennung beider Fraktionen vermieden.

Die in den Figuren 1 bis 3 gezeigte Zentrifugationskammer besteht aus einer Probenkammer 7, die bevorzugt aus einem Kunststoff-Spritzteil besteht. Die Probenkammer 7 ist abdichtend mittels Dichtungsringen 5, 6 auf einem Objektträger 2 aufgesetzt, wobei sie mittels Klemmbügeln 3, welche in Halterungen 4 an der Seite einer Trägerplatte 1 angeordnet sind, gegen diese Trägerplatte 1, auf der der Objektträger 2 angeordnet ist, festgeklemmt wird. Die linke Bohrung der Probenkammer 7 besteht aus einer oberen, kleineren Bohrung 9 und einer damit fluchtenden unteren, größeren Bohrung 8, wobei zwischen beiden Bohrungen 8, 9 ein Ansatz besteht.

Neben der abgesetzten Bohrung 8, 9 ist parallel im Abstand eine weitere Bohrung 10 angeordnet, in deren Bohrungswandung im Abstand oberhalb der Ebene des Objektträgers 2 eine Durchgangbohrung 17 vorgesehen ist, die zur waagerechten Überlaufbohrung 14 fluchtet und zu deren Herstellung dient.

Von Übergang der kleineren Bohrung 9 in die größere Bohrung 8 ausgehend ist eine Überlaufbohrung 14 angeordnet, welche in die rechte Bohrung 10 mündet. In der Probenkammer 7 ist in eine Gewindebohrung eine Klemmschraube 15 eingesetzt, welche mit ihrer Spitze in den lichten Querschnitt der Überlaufbohrung 14 ragt. Durch mehr oder weniger Herausdrehen oder Hereindrehen der Klemmschraube 15 kann somit der Querschnitt der Überlaufbohrung 14 eingestellt werden.

Vor Beginn des Zentrifugiervorgangs wird in die linke Bohrung 8, 9 zunächst das Trennmedium (z.B. Plasma steril) (vergl. Figur 4) eingefüllt, das von der Probensuspension überschichtet wird.

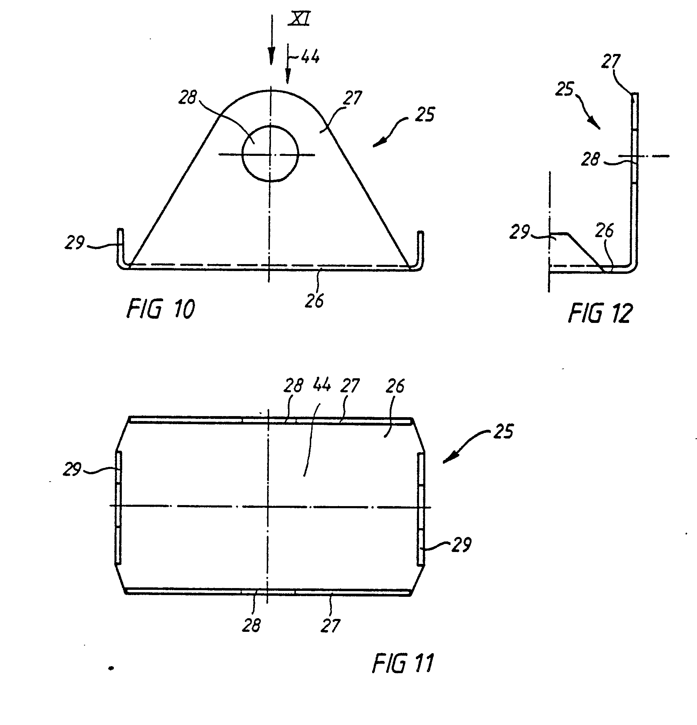

Gemass der späteren Beschreibung in Verbindung mit den Figuren 10—12 wird erläutert werden, daß die gesamte Probenkammer 7 in ein Schaukelgehänge mit einer Zentrifuge eingesetzt wird, so daß während des Zentrifugiervorgangs die Ebene des Objektträgers 2 vertikal steht und die Längsachsen der Bohrungen 8, 9 bzw. 10 horizontal ausgerichtet sind. Die Probenkammer 7 wird hierbei bezüglich der Darstellung in Figur 1 und 90° gedreht in einen Topfrotor eingesetzt, so daß die linke vertikale Wand der Bohrung 9 parallel zur Bodenfläche und die rechte, vertikale Wand der Bohrung 10 parallel zur Deckelfläche des Topfrotors zu liegen kommen. Die Zentrifugalkraft wirkt dann gemäss das Darstellung in Figur 1 in Richtung der Längsachse der Bohrungen 9, 10 nach unten in Richtung auf den am distalen Ende angeordneten Objektträger 2(Pfeilrichtung 44).

Die Figur 1 zeigt schematisiert die Trennung der Probe in eine proximale Fraktion 12 und eine distale Fraktion 16, während des Zentrifugiervorganges, wobei hinsichtlich der distalen Fraktion 16 die Epithelzellen der Probensuspension in das Trennmedium eindiffundieren und aufgrund der Zentrifugalkraft an der unteren Stirnseite der Bohrung 8 auf den Objektträger 2 aufsedimentiert werden.

Zur Abtrennung der proximalen Fraktion 12 (Überstand) von der distalen Fraktion während des Zentrifugationsvorganges wird die proximale Fraktion 12 allmählich aufgrund der in Längsrichtung der Bohrung 9 nach unten gerichteten Zentrifugalkraft in die Bohrung 8 gedrückt, wobei ein Überdruck ensteht, der durch die Überlaufbohrung 14, welche am Absatz 11 der Bohrung 8 mündet, entlastet wird.

Die proximale Fraktion 12 wird daher langsam in Pfeilrichtung 13 durch die Überlaufbohrung 14 strömen und in die benachbarte Bohrung 10 einfliessen. Je nach Dauer des Zentrifugiervorges und je nach Einstellung der Klemmschraube 15 wird mehr oder weniger Zeit benötigt, bis die gesamte proximale Fraktion 12 in die benachbarte Bohrung 10 eingeflossen ist.

Hier erfolgt während der Fortsetzung des Zentrifugationsvorganges eine Aufsedimentation der proximalen Fraktion 12 auf das Beobachtungsfeld des Objektträgers 2 an der Stirnseite der Bohrung 10.

Mit einem einzigen Zentrifugationsvorgang wurde also die Aufsedimentierung der proximalen und der distalen Fraktion auf einen Objektträger 2 durchgeführt, wobei das umständlich Trennen der beiden Fraktionen durch Abpipettierung und nachfolgende Präparation entfiel.

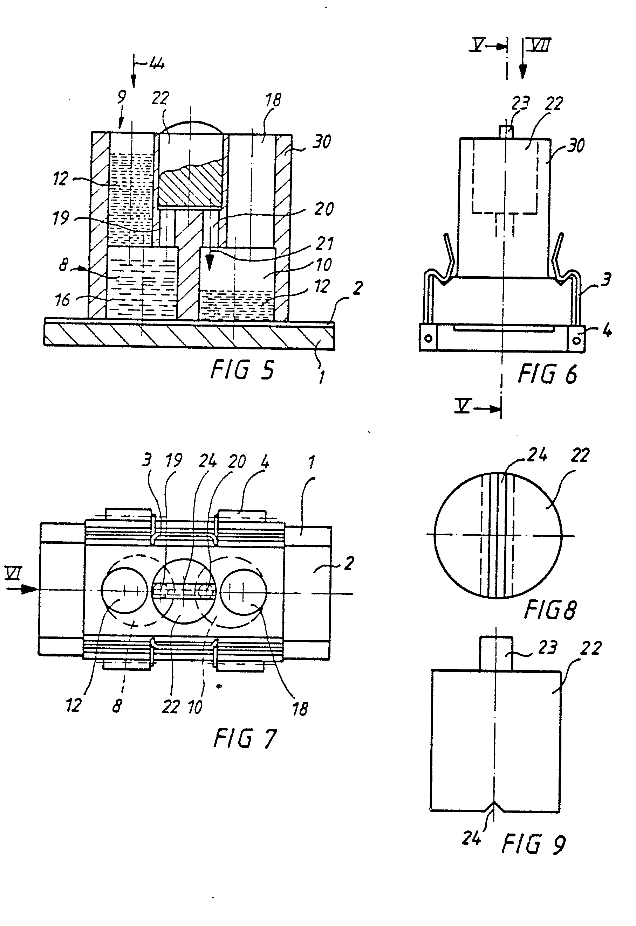

Die Figuren 5—9 zeigen eine weitere Ausführungsform einer Probenkammer 30, wobei die mit dem Ausführungsbeispiel in den Figuren 1 bis 3 übereinstimmenden Teile mit den gleichen Bezugszahlen versehen wurden.

Unterschiedlich ist, daß statt einer Klemmschraube ein Verschlußstück 22 verwendet wird, das in den Figuren 8 und 9 näher dargestellt ist.

Die Bodenfläche des Verschlußstückes 22 weist einen radial durchgehenden Verbindungskanal 24 auf, der als Kerbe mit konischem Querschnitt ausgebildet ist. Das Verschlußstück ist hierbei axial in der Probenkammer 30 in einer zugeordneten Bohrung verschiebbar, wobei der Verbindungskanal 24 des Verschlußstückes 22 die Verbindung zwischen einer linken Überlaufbohrung 19, welche in die größere Bohrung 8 mündet, und einer rechten Überlaufbohrung 20, welche in die rechte Bohrung 10 mündet, dargestellt.

Je größer die Zentrifugalkraft ist, desto stärker wird das Verschlußstück 22 mit seinem Verbindungskanal 24 auf die ebene Fläche zwischen den Verbindungsbohrungen 19, 20 gepresst und desto geringer ist der Durchlaßquerschnitt. Das Verschlußstück 22 kann aus einem elastischen Kunststoff gefertigt sein, der sich unter dem Einfluß der Zentrifugalkraft verformt, so daß hierdurch eine Verminderung des Querschnittes gegeben ist. Mit einem Griffteil 23 kann das Verschlußstück 22 aus der Probenkammer 30 entnommen werden.

Während des Zentrifugalvorganges erfolgt ebenfalls unter dem Einfluß der in Pfeilrichtung 44 wirkenden Zentrifugalkraft eine Auftrennung der in der linken Bohrung 8, 9 eingefüllten Probensuspension in eine distale Fraktion 16 und eine darüber geschichtete, proximale Fraktion 12. Nachdem die Überlaufbohrung 19 wiederum von dem Absatz 11 zwischen den Bohrung 8, 9 ausgeht, wird aufgrund der herrschenden Zentrifugalkraft der auf die proximale Fraktion 12 wirkende Druck über die Überlaufbohrung 19, den Verbindungskanal 24 und die Überlaufbohrung 20 abgeleitet, so daß die proximale Fraktion 12 in Pfeilrichtung 21 in die rechte Bohrung 10 einfließt und dort auf dem Objektträger 2 aufsedimentiert wird.

Die rechte Bohrung 10 ist hierbei als angesetzte Bohrung mit einer versetzt angeordneten kleineren Bohrung 18 ausgebildet. Hinsichtlich der Figuren 6 und 7 gelten für gleiche Teile die gleichen Bezugszeichen, wie sie in Verbindung mit den Figuren 1—3 verwendet wurden.

Die Figuren 10 bis 12 zeigen ein Schaukelgehänge, wie es zur Verwendung mit den Probenkammern 7, 30 nach den Figuren 1—3 bzw. 5—7 verwendet wird. Das dort gezeigte Schaukelgehänge 25 besteht aus einer Trägerplatte 26 an der ein seitlicher und gegenüberliegender Rand 29 angeordnet ist. Um 90° hierzu versetzt setzen am Rand der Trägerplatte 26 Schenkel 27 an, welche jeweils von einer Bohrung 28 durchsetzt sind. Die einander gegenüberliegenden Bohrungen 28 werden in nicht näher dargestellter Weise von einem Trägerzapfen durchsetzt, der am Zentrifugenmotor befestigt ist. Die Probenkammer 7, 30 wird mit ihrer Trägerplatte 1 auf die Trägerplatte 26 des Schaukelgehänges 25 aufgesetzt, wobei der erhöhte Rand 29 und die seitwärts hochstehenden Schenkel 27 ein Herabfallen der Probenkammer 7, 30 von dem Schaukelgehänge 25 vermeiden. Während des Zentrifugiervorganges schwingt das Schaukelgehänge 25 mit seiner Bohrung 28 um den nicht näher dargestellten Zapfen, so daß die Trägerplatte 26 senkrecht steht, ebenso wie die Trägerplatte 1 und der parallel hierzu angeordneten Objektträger 2 der Probenkammer 7, 30.

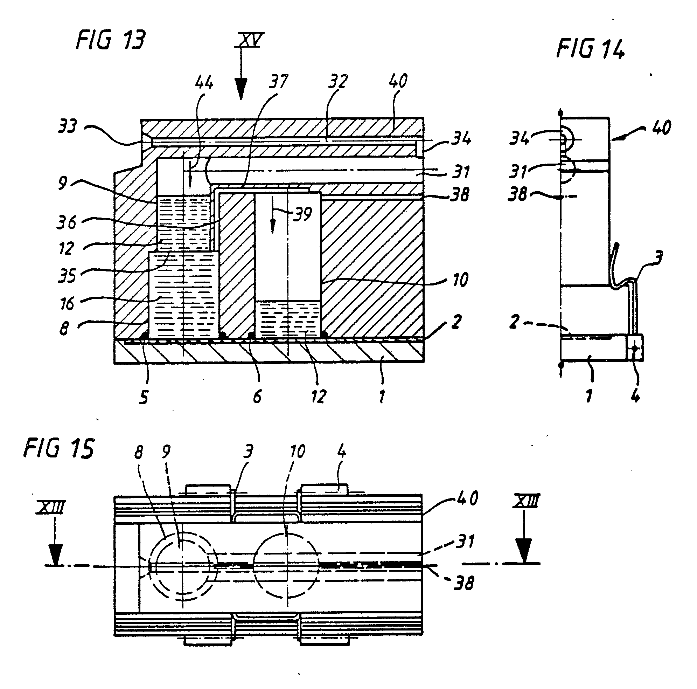

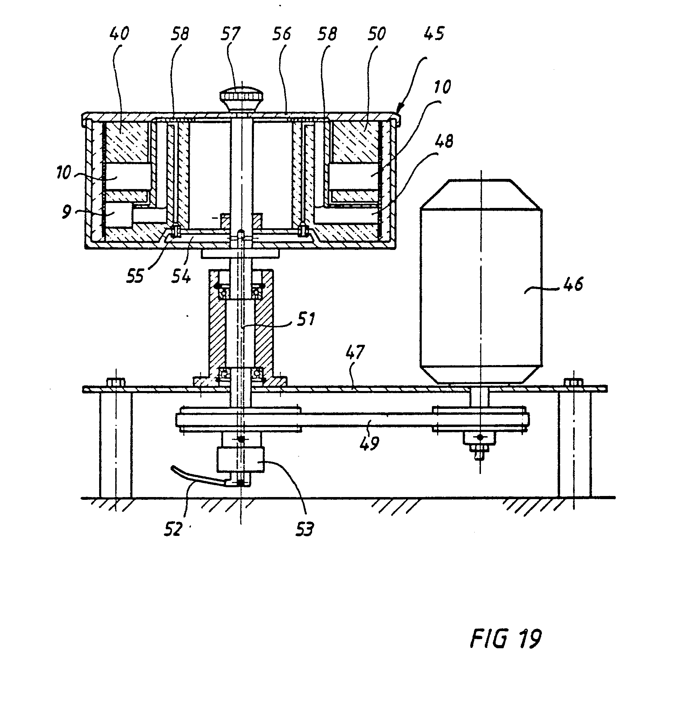

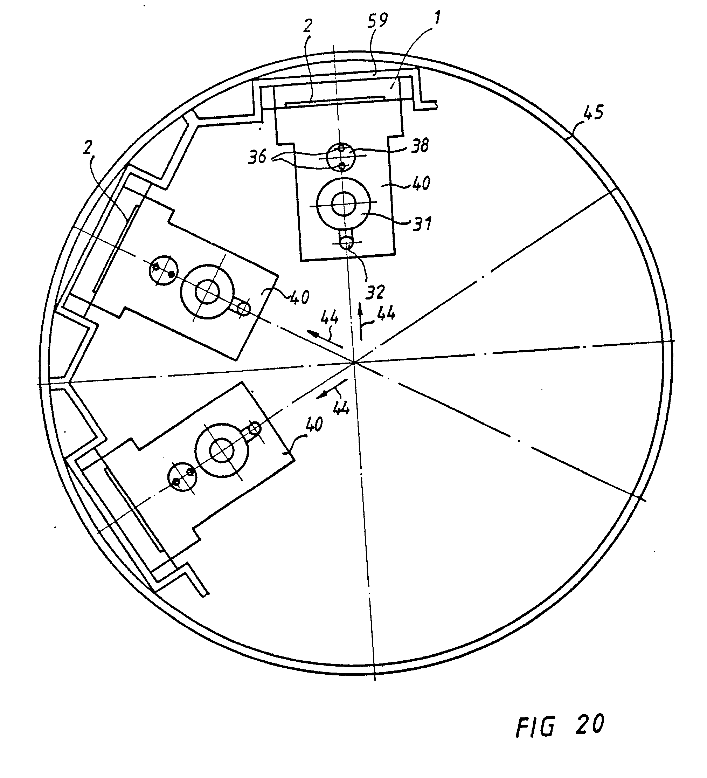

Die in den Figuren 13 bis 15 und 16 bis 18 beschriebenen Probenkammern 40, 50 sind zur Verwendung mit einem in den Figuren 19 und 20 gezeigten Topfrotor 45 bestimmt. Die Einbaulage dieser Probenkammer 40, 50 geht aus Figur 19, hervor. Hierbei ist wesentlich, daß ein Schaukelgehänge fehlt und stattdessen diese Probenkammern 40, 50 unmittelbar liegend in dem Topfrotor 45 eingesetzt werden. Sowohl in der Ruhelage als auch in der Arbeitslage wird daher die Lage der Probenkammern 40, 50 nicht verändert, d.h., die Trägerplatte 1 mit dem dahinter angeordneten Objektträger 2 steht immer senkrecht und die linke, in den Abbildungen 13 bis 15 und 16 bis 18 gezeigte, vertikale Wand der Bohrung 9 bildet die Bodenfläche der Probenkammer 40, 50.

Die in den Figuren 13 bis 15 dargestellte Probenkammer 40 weist wiederum eine Trägerplatte 1 mit einem darauf angeordneten Objektträger 2 auf, wobei die Probenkammer 40 mit Hilfe der vorher beschriebenen Halterung 4 und den Klemmbügeln 3 gegen den Objektträger und gegen die Trägerplatte 1 gepresst wird.

Die Befüllung der die Probensuspension und die Plasma-Steril-Lösung aufnehmenden Bohrung 8, 9 erfolgt über einen Einfüllstutzen 31, der gemäss Figur 19 in Einbaulage senkrecht nach oben mit seiner Mündung weist.

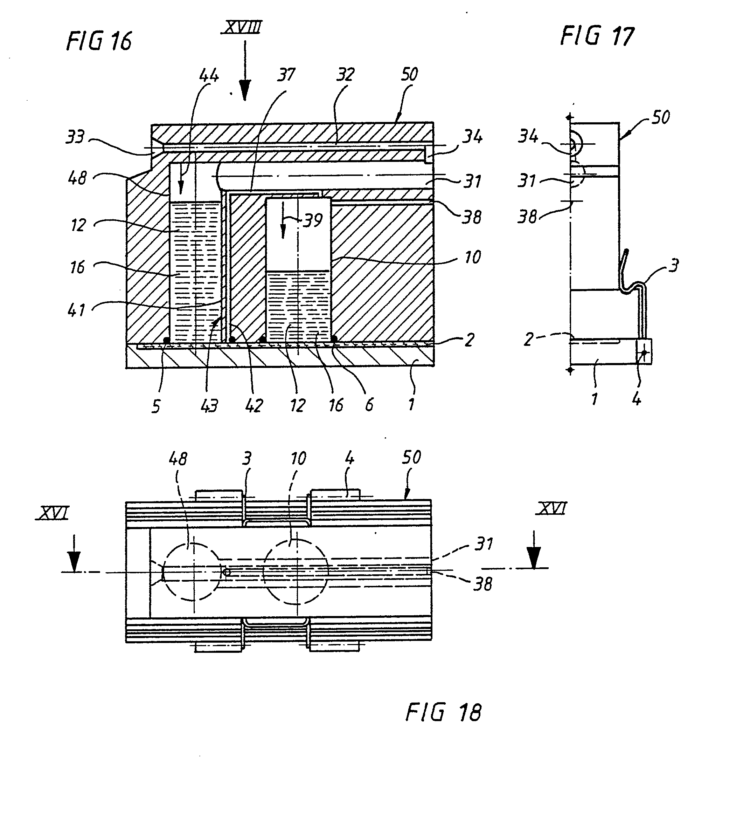

Während des Zentrifugiervorgangs bildet sich wiederum unter dem Einfluß der Pfeilrichtung 44 wirkenden Zentrifugalkraft gemäss Figur 13 eine proximale Fraktion 12 und eine distale Fraktion 16 in den übereinanderliegenden und zueinander fluchtenden Bohrungen 8, 9 der linken Kammer. Zur Abtrennung der proximalen Fraktion 12 von der distalen Fraktion 16 während des Zentrifugiervorgangs wird Druckluft verwendet. Die Druckluft wird gemäss Figur 19 über einen Druckluftschlauch 52 und einen Druckluftanschluß 53 in die Rotorwelle 51 der Topfzentrifuge 45 eingespeist, wo sie über einen am Boden des Topfrotors 45 angeordneten Verteilerkanal 54 in Anschlußstücke 55 gelangt, die in eine zugeordnete, konische Aufnahmebohrung (Anschlußstück 33) der Figur 13 bzw. Figur 16 eingreifen. Vom Anschlußstück 33 ausgehend (Figur 13 bzw. Figur 16) wird die Druckluft über den Druckkanal 32 zur Oberseite der Probenkammer 40, 50 geleitet. Die Überleitung in die Mündung des Einfüllstutzens 31 erfolgt dadurch, daß ein einseitig offenes Verbindungsstück 34 zwischen der Mündung des Druckluftkanals 32 und der Mündung des Einfüllstutzens 31 vorhanden ist, und die genannten Teile luftdicht von einer elastischen Platte 58 abgedeckt werden, die an der Unterseite des Deckels 56 des Topfrotors 45 angeordnet ist. Hierdurch wird also eine luftschlüssige Verbindung zwischen dem Druckluftschlauch 52 und dem Einfüllstutzen 31 geschaffen. Die Druckluft wirkt dann in Pfeilrichtung 44 auf die Oberfläche der proximalen Fraktion 12, die aufgrund des Druckunterschiedes über einen Verbindungskanal 36 und ein Teilstück 37 in Pfeilrichtung 39 in die rechte Bohrung 10 eingeleitet wird. Die Bohrung 38 mündet neben der elastischen Platte 58 gemäss Figur 19 und dient sur Entlüftung.

Mit dem Ausführungsbeispiel einer Probenkammer 50 gemäss den Figuren 16—18 wird eine pneumatisch beaufschlagte Überlaufkammer mit bis zum Objektträger 2 führenden Überlaufkanal (Verbindungskanal 42) und Entlüftung (Bohrung 38) vorgeschlagen.

Gleiche Teile, wie beim Ausführungsbeispiel nach den Figuren 13 bis 15 sind mit gleichen Bezugszeichen bezeichnet.

Die Besonderheit dieses Ausführungsbeispieles ist, daß statt einer abgesetzten Bohrung 8, 9 im Ausführungsbeispiel der Figuren 13 bis 15 eine durchgehende Bohrung 48 vorgesehen ist, und daß parallel und im Abstand zu dieser Bohrung 48 der Verbindungskanal 42 im Material der Probenkammer 50 verläuft. Es ist nun möglich, mit einer Nadel die Wand 41 der Bohrung 48 anzustechen; dies kann beispielsweise in Höhe des Pfeiles 43 geschehen, so daß beliebig vorher bestimmbaren Höhen (Abstand vom Objektträger 2) die Verbindung zu dem Verbindungskanal 42 aus der Bohrung 48 geschaffen werden kann. Hiermit ist es möglich, die Restflüssigkeit in der Bohrung 48 zu variieren.

Der in Abbildung 16 senkrecht verlaufende Verbindungskanal 42 geht in ein horizontales Teilstück 37 über und mündet in die rechte Bohrung 10. Eine als Entlüftung der Bohrung 10 funktionierende Bohrung 38 mündet neben der plastischen Platte 58 gemäss Figur 19.

Mit der freien Wahl der Fraktionierungsgrenze durch Anstechen der Verbindungskanals 42 in Pfeilrichtung 43 ist es möglich, sowohl in der linken Bohrung 48 als auch in der rechten Bohrung 10 eine distale une proximale Fraktion 12, 16 zu erhalten, wobei die beiden Bohrungen verschieden schwere Fraktionen beinhalten.

Die Figur 19 zeigt auch schematisiert den Einbau einer nach den Figuren 16—1 gezeigten Probenkammer 50.

In der Draufsicht nach Figur 20 wird die Probenkammer 40 oder 50 in der in Figur 19 gezeigten Einbaulage in einen vertikalen Rotorrahmen 59 eingesetzt und darin formschlüssig festgehalten. Es wird dann der Deckel 56 mit der darunterliegenden elastischen Platte 5 aufgesetzt, so daß der Druckluftschlauch 52 luftschlüssig mit dem Einfüllstutzen 31 der Probenkammer 40 oder 50 verbunden ist. Der Deckel 56 wird mit dem Schließknopf 57 festgespannt. Der Deckel 56 mit elastischer Platte 58 dient gleichzeitig als Spannmittel für die Probenkammer 40 oder 50.

Es wird sodann die Zentrifuge eingeschaltet, wobei der Motor 46 auf einer Trägerplatte 47 montiert ist und über einen Treibriemen 49 die Rotorwelle 51 antreibt.

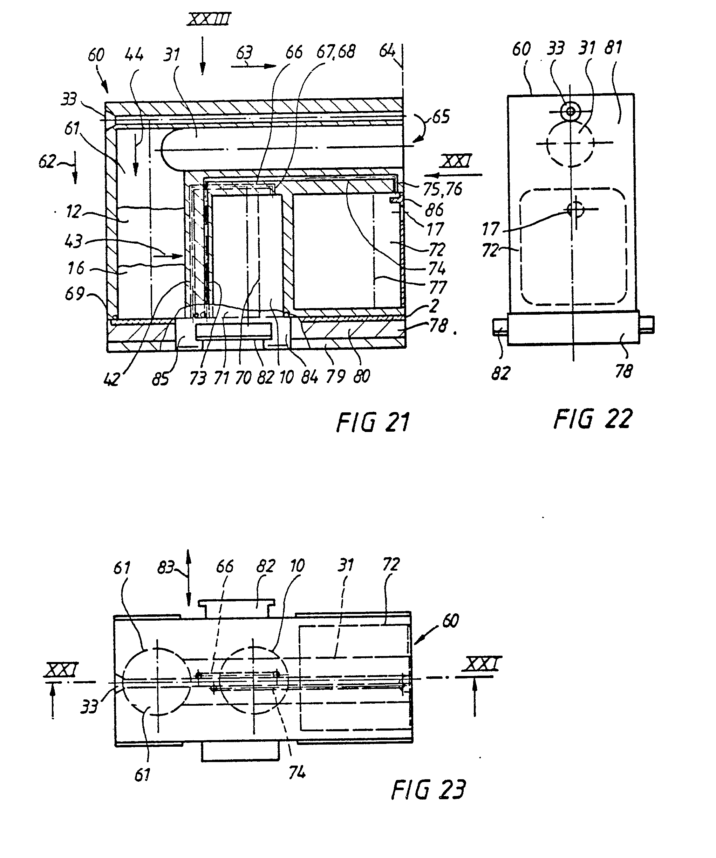

Das Ausführungsbeispiel nach den Figuren 21—23 zeigt eine Weiterentwicklung des Ausführungsbeispieles nach Figur 16.

Es ist eine große Bohrung 61 vorhanden, in der in der vorher beschriebenen Weise während des Zentrifugiervorganges sich die proximale Fraktion 12 und die distale Fraktion 16 absetzt. Nach erfolgter Trennung wird während des Zentrifugiervorgangs Pressluft in den Kanal 33 eingeleitet, die in Pfeilrichtung 63 in den Kanal einströmt, dort von dem in Figur 19 dargestellten Deckel 56 in Pfeilrichtung 65 umgelenkt wird und über den Kanal 31 in Pfeilrichtung 44 in die Bohrung 61 einströmt. Dort wird die Probenflüssigkeit über den Kanal 42 verdrängt und fließt in die zweite Bohrung 10. Die Erweiterung dieses Ausführungsbeispieles gegenüber dem Ausführungsbeispiel nach Figur 16 liegt nun darin, daß das Teilstück 37 nach Figur 16 entfällt und daß stattdessen ein Teilstück 66 vorgesehen ist, was in ein weiteres Teilstück 67 des Kanals übergeht. Wichtig ist, daß die Mündung 68 des Teilstücks 67 in die kleine Bohrung 10 bei aufrechtstehender Probenkammer, d.h. wenn die Probenkammer auf den Bodenfläche 69 steht, oberhalb des sich bei stehender Probenkammer bildenden Flüssigkeitsspiegels 70 liegt, so daß auf jeden Fall vermieden wird, daß Probenflüssigkeit 71 von der zweiten Bohrung 10 über die Teilstücke 66, 67 und den Kanal 42 zurück in die erste Bohrung 61 fließt.

Ein weitere Erweiterung des Erfindungsgedankens liegt in der Anordnung einer dritten Bohrung 72. Die Bohrung 72 ist in Serie zu der zweiten Bohrung 10 geschaltet. Wichtig ist hierbei, daß ein Kanal 73 unterhalb des Flüssigkeitsspiegels 70 bei stehendem Probengefäss in die zweite Bohrung 10 mündet. Die Mündung bei dem Ausführungsbeispiel nach Figur 21 ist unmittelbar über dem Objektträger 2 gezeigt, wo ein geringes Spiel von 2/10 mm noch vorhanden ist; diese Mündung kann aber irgendwo im Bereich (z.B. bei Pfeil 43) des Zentrifugenkanals 73 eingebracht sein.

Der Kanal 73 setzt sich in einen vertikalen Kanal 74 fort, der in einen weiteren, horizontalen Kanal 75 übergeht, der in die Bohrung 72 mündet.

Wesentlich ist hierbei, daß die Mündung 76 des Kanals 75 oberhalb des Flüssigkeitsspiegels 77 liegt, so daß auch aus dieser dritten Bohrung 72 keine Flüssigkeit in die zweiten Probenkammern zurückfließen kann, wenn die Zentrifuge steht.

Die Anordnung einer dritten Bohrung 72 hinter der zweiten Bohrung 10 und deren Verbindung über die Kanäle 73, 74, 75 bedeutet einen wesentlichen Fortschritt in der Zytologie, weil mit einem solchen—insgesamt drei Bohrungen enthaltenden-Probengefäss ein Färbevorgang während des Zentrifugiervorganges vorgenommen werden kann.

Dies geht folgendermassen:

Ausgehend von der proximalen 12 und distalen Fraktion 16, die sich in der ersten Bohrung 61 bildet, wird nachfolgend Druckluft über den Kanal 33 eingeleitet, die in Pfeilrichtung 44 die gesamte Flüssigkeit der Bohrung 61 über den Kanal 42, 66, 67, 68 in die zweite Bohrung 10 verdrängt. Die Zentrifuge wird nun angehalten und die Flüssigkeit nimmt eine Lage mit dem Flüssigkeitsspiegel 70 ein. Es wird nun wieder Druckluft über den Kanal 33 eingeleitet, die nun durch die leere Probenkammer 61 strömt, dort über den Kanal 42, 66, 67, 68 in die zweite Bohrung 10 einströmt, die Probenflüssigkeit unter Druck setzt und über den Kanal 73, 74, 75 in die dritte Bohrung 72 verdrängt.

Die Anordnung einer dritten Bohrung 72 ist wichtig, denn es kann- nachdem die beiden ersten Probenkammern freigemacht wurden—ein Färbevorgang stattfinden. Hierzu wird die Zentrifuge angehalten, der Deckel wird geöffnet und über den Einfüllstutzen 31 wird in die erste Bohrung 61 ein paar Tropfen einer Färbeflüssigkeit gegeben. Nachdem die Probenflüssigkeit eingefüllt wurde, wird die Zentrifuge eingeschaltet und die Probenflüssigkeit wird gegen die Objektträgerfläche der ersten Bohrung 61 gepresst und färbt die dort lagernden Epithelzellen ein. Es wird eine bestimmte Einwirkungszeit abgewartet, dann wird Preßluft über den Kanal 33 gegeben, welche in Pfeilrichtung 44 auf die in der Nähe der Objektträgerfläche lagernde Färbeflüssigkeit einwirkt, die nun über den Kanal 42, 66, 67 in die zweite Bohrung 10 verdrängt wird und das dortige Objektträgerfeld einfärbt. Nach erfolgter Einwirkungszeit wird die Zentrifuge angehalten, und die Färbeflüssigkeit fließt nach unten auf die Bodenfläche, die parallel zum Flüssigkeitsspiegel 70 liegt. Der Kanal 73 von der Bohrung 72 mündet im Bereich dieser Bodenfläche in die Bohrung 10 so daß während des Stillstehens der Probenkammer 60 und während der Einleitung von Preßluft diese in Pfeilrichtung 44 in die erste Bohrung 61 einströmt, dort über den Kanal 42, 66, 67 in die zweite Bohrung 10 strömt und dort die Färbeflüssigkeit über die Mündung des Kanals 73 an der Objektträgerfläche in den Kanal 73 hineingepresst und über den Kanal 74, 75 und die Mündung 76 in die dritte Bohrung in die dritte Bohrung 72 drückt. In der dritten Bohrung 72 sammelt sich eine Abfallflüssigkeit, die aus einem Gemisch aus Probenflüssigkeit und aus Färbeflüssigkeit besteht. Um ein Auströmen der Flüssigkeit aus der der Entlüftung dienenden Durchgangsbohrung 17 zu vermeiden, ist zwischen der Mündung 76 des Kanals 75 und der Durchgangsbohrung 17 ein Vorsprung 86 angeordnet.

Nach erfolgtem Färbevorgang kann in analoger Weise noch weitere Flüssigkeit eingegeben werden, z.B. eine Spülflüssigkeit, eine Fixierflüssigkeit und ähnliches, wobei sich sämtliche verbrauchten Lösungen dann nach erfolgten, durchgeführten Arbeitsvorgangen in der Bohrung 72 sammeln.

Durch die Serienschaltung von mehreren, hintereinanderliegenden Bohrung 61, 10, 72 und durch die sinnvolle Verbindung dieser Bohrungen 61, 10, 72 mit Hilfe von Kanälen werden also die Objektträgerflächen des Objektträgers 2 der Bohrungen 61, 10 (d.h., also die Untersuchungsfläche und die Kontrollfläche des Objektträgers) mit verschiedenen Behandlungsflüssigkeiten behandelt, ohne daß jemals die gesamte Probenkammer 60 aus dem Rotor der Zentrifuge herausgenommen werden muss.

In der Figur 21—23 ist eine Halterung gezeigt, die es ermöglicht, daß die gesamte Bodenplatte 78, die aus zwei Teilplatten 79, 80 besteht, einfach von dem Oberteil 81 abgenommen werden kann. Hierzu ist es möglich, durch Verschieben der Probenkammer 60 eines Schiebers 82 mit einem einzigen Handgriff die Bodenplatte 78 von dem Oberteil 81 zu lösen, so daß der Objektträger 2 unmittelbar entnommen werden kann. Der Schieber ist hierbei in den Pfeilrichtungen 83 verschiebbar und arbeitet mit entsprechenden Haltenasen 84, 85 des Oberteils zusammen.

In der Figur 23 ist zur besseren Verdeutlichung des Erfindungsgedankens das Grundprinzip schematisiert dargestellt, wie die drei Bohrungen nach der Figur 21 hintereinandergeschaltet sind. Ebenso sind die Flüssigkeitsströme gezeigt und die Mündungen der Kanäle. Gestrichelt bei der zweiten Bohrung 91 ist gezeigt, daß auch ein zweiter Kanal 73 angeordnet sein kann. Der in der zweiten Bohrung 91 durchgezogen gezeichnete Kanal 73 ist dafür gedacht, daß die Probenflüssigkeit nur dann aus der zweiten Bohrung 91 entnommen wird, wenn die Zentrifuge angehalten still steht, so daß die Flüssigkeit auf die Bodenfläche fließt und dort den Flüssigkeitsspiegel 70 bildet, so daß sie über den Kanal 73 aus der zweiten Bohrung 91 in die dritte Bohrung 93 eingeleitet werden kann. Will man die Überleitung von der zweiten in die dritte Bohrung aber während des Zentrifugiervorgangs durchführen, dann wählt eine Fließverbindung entsprechend dem gestrichelt gezeichneten Kanal 73', der in der Nähe des Objektträgers 2 endet. Die in die zweite Bohrung 91 einströmende Flüssigkeit wird zwar nach und nach unter Einwirkung der Druckluft sofort über diesen Kanal 73' in die dritte Bohrung 93 verdrängt werden, man kann den Querschnitt des Kanals 73' jedoch so gering wählen, daß die geforderte Einwirkungszeit die Probenflüssigkeit auf den Objektträger 2 in der zweiten Bohrung 91 beibehalten wird.

D.h., man wählt den Querschnitt beispielsweise so, daß erst nach etwa 2 Minuten der Inhalt der zweiten Bohrung 91 vollständig in die dritte Bohrung 93 eingeleitet wurde, so saß eine Einwirkungszeit von 2 Minuten bei der zweiten Bohrung 91 gewährleistet ist.

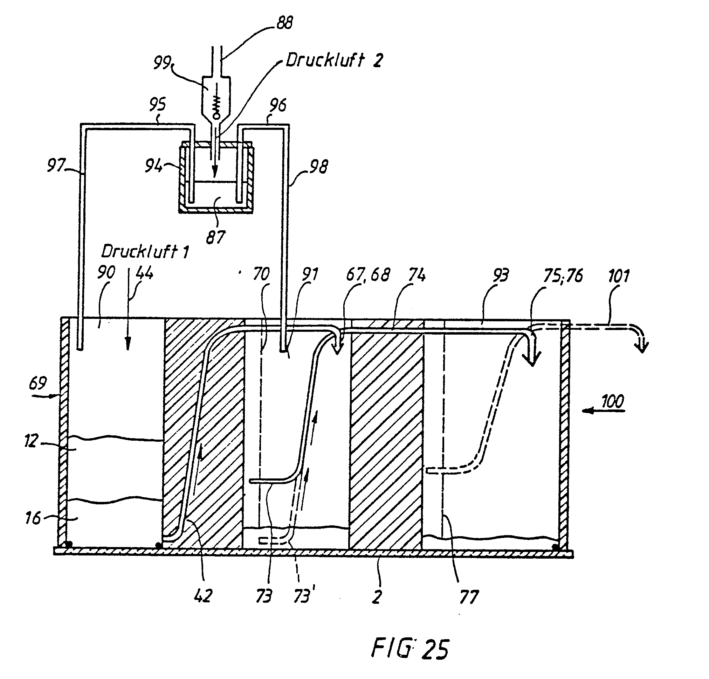

Figur 25 zeigt ein weiteres, abgewandeltes Ausführungsbeispiel, wo gezeigt ist, daß mit einem weiteren Reservoir (Farbreservoir 94 für Farbflüssigkeit) ohne Anhalten der Zentrifuge und Öffnen des Zentrifugendeckels sofort ein Färbevorgang durchgeführt werden kann, sofern die beiden Probenkammern 61 und 10 vorher entleert wurden, wie es anhand der Fig. 21—23 erläutert wurde. Wenn man über einen zweiten Druckluftanschluß 88, der etwa parallel zu dem Kanal 33 in der Probenkammer angeordnet ist, Druckluft in ein Farbreservoir 94 einpresst, dann wird über den Überlauf 95, 96 die Farbe 87 aus dem Farbreservoir 94 verdrängt und über die Leitungen 97, 98 sofort in die Bohrungen 90, 91 geleitet. Es entfällt somit das Anhalten der Zentrifuge und das Einbringen der Farbe 87 zunächst in die erste Bohrung 61 und die Überleitung in die zweite Bohrung 91. Es wird vielmehr die Farbe 87 bei laufender Zentrifuge parallel in beide Bohrungen 91, 92 verteilt.

Es wird damit auch eine Verkürzung der Verfahrenszeit erreicht.

Sobald die Färbung vorgenommen wurde, kann in der vorher beschriebenen Weise die Farbflüssigkeit aus den Bohrungen 90, 91 entfernt werden, indem auf die erste Bohrung 90 die Druckluft in Pfeilrichtung 44 aufgegeben wird, so daß die Farbe 87 von der ersten Bohrung 90 über den Kanal 42 in die zweite Bohrung 91 übergeleitet wird. Wenn die erste Bohrung 42 entleert ist, wird auch die zweite Bohrung 91 über den Kanal 73, 74, 75 entleert, und sämtliche Farbe 87 fließt in die dritte Bohrung 93.

In Serie hinter der dritten Bohrung 93 können noch weitere—nicht näher dargestellte—Bohrungen angeordnet sein. Als Beispiel ist noch eine Fließverbindung von der dritten Bohrung 93 in eine nicht näher dargestellte Bohrung in Form eines weiteren Kanals 101 gezeigt.

Damit während der Einleitung von Drukluft in Pfeilrichtung 44 in unzulässiger Weise über die Leitungen 97, 98 Druckluft in das Farbreservoir 94 zurückgedrückt wird, und über den Druckluftanschluß 88 entweicht, ist dort ein Rückschlagventil 99 angeordnet.

In analoger Weise kann das Beispiel nach Fig. 25 beliebig viele Behandlungsflüssigkeits-Reservoirs aufweisen, z.B. ein zweites Reservoir für Spülflüssigkeit oder ein drittes Reservoir für eine Fixierflüssigkeit und so weiter. Wesentlich bei dieser Erweiterung ist, daß jedem Reservoir 94 ein eigener Druckluftanschluß mit einem zugeordneten Rückschlagventil zugeordnet werden muß, damit das geordnete Herausfließen aus dem jeweiligen Reservoir zum richtigen Zeitpunkt gewährleistet ist.

Das vorgeschlagene System hat also den Vorteil, daß die Probenkammer 100 ständig in der Zentrifuge verbleibt und der Färbevorgang unter Zentrifugalkraft in der Zentrifuge selbst erfolgt und teure, sowie kostspielige und ungenaue automatische Färbevorrichtungen vermieden werden. Vor allem wird verhindert, daß Epithelzellen bei externen Färbeverfahren von den Objektträgerflächen abgespült werden, was als großer Nachteil bei den bisher bekannten Färbemethoden zu werten ist.

Es kann aber nicht nur der Färbevorgang nach der vorliegenden Erfindung automatisiert werden, sondern auch der Fixiervorgang und der Spülvorgang und dergleichen mehr.

Wesentlich ist ferner, daß die gesamte Probenkammer 7, 80, 40, 60, 100 aus einem relativ billigen Kunststoff-Spritzguß besteht, einfach herzustellen ist und daß sogar die O-Ringe, die in den Zeichnungen noch angegeben sind, durch angespritzte Lippendichtungen ersetzt werden können, so daß es möglich ist, eine solche Probenkammer als Einwegartikel mit niedrigen Einkaufskosten herzustellen.

**Patentansprüche**

1. Zentrifugationskammer zur zytodiagnostischen Präparation von in einer Suspensionsprobe enthaltenen Epithelzellen, bestehend aus einer Trägerplatte (1) und einem darauf befestigen Objektträger (2), auf dessen Seite abgedichtet eine Probenkammer (7, 30, 40, 50, 60, 100) aufsitzt, welche mehrere parallel und im Abstand nebeneinander angeordnete und mit Probenflüssigkeit füllbare Bohrungen (8, 9, 10; 9, 19, 18; 48, 10; 61, 10, 72; 90, 91, 93) aufweist, deren stirnseitige Öffnungen jeweils dichtend auf dem Objektträger (2) aufsitzen, dadurch gekennzeichnet, daß zwischen diesen Bohrungen (8, 9, 10; 9, 19, 18; 48, 10; 61, 10, 72; 90, 91, 93; 101) jeweils eine Fließverbindung in Form eines Kanals (14, 15; 19, 20, 24; 36, 37; 42; 73, 74, 75, 76) angeordnet ist, wobei diese Fließverbindung aus einer im Querschnitt veränderbaren Überlaufbohrung (14; 19, 20, 24) besteht und wobei die eine Bohrung (8, 9) in eine Bohrung (8) größeren Durchmessers und in eine mit dieser (8) fluchtenden Bohrung (9) kleineren Durchmessers aufgeteilt ist, und die Überlaufbohrung (14; 19, 20, 24) vom Ansatz der größeren (8) in die kleinere Bohrung (9) ausgeht (Fig. 1—3 und 5—9).

2. Zentrifugationskammer nach Anspruch 1, dadurch gekennzeichnet, daß zur Veränderung des Querschnitts der Überlaufbohrung (14) eine Klemmschraube (15) vorgesehen ist (Fig. 1—3).

3. Zentrifugaktionskammer nach Anspruch 1, dadurch gekennzeichnet, daß zur Veränderung des Querschnitts der Überlaufbohrung (19, 20, 24) ein axial in Längsrichtung der Bohrungen (8, 9, 19) verschiebbares Klammstück (22) vorgesehen ist, dessen eine Stirnfläche einen radialen Verbindungskanal (24) aufweist, der die Verbindung zwischen den beiden Überlaufbohrungen (19, 20) bildet (Fig. 5—9).

4. Zentrifugationskammer zur zytodiagnostischen Präparation von in einer Suspensionsprobe enthaltenen Epithelzellen, bestehend aus einer Trägerplatte (1) und einem darauf befestigen Objektträger (2), auf dessen Seite abgedichtet eine Probenkammer (7, 30, 40, 50, 60, 100) aufsitzt, welche mehrere parallel und im Abstand nebeneinander angeordnete und mit Probenflüssigkeit füllbare Bohrungen (8, 9, 10; 9, 19, 18; 48, 10; 61, 10, 72; 90, 91, 93) aufweist, deren stirnseitige Öffnungen jeweils dichtend auf dem Objektträger (2) aufsitzen, dadurch gekennzeichnet, daß zwischen diesen Bohrungen (8, 9, 10; 9, 19, 18; 48, 10; 61, 10, 72; 90, 91, 93, 101) jeweils eine Fließverbindung in Form eines Kanals (14, 15; 19, 20; 24; 36, 37; 42; 73, 74, 75, 76) angeordnet ist, wobei zur Überleitung von Flüssigkeit von einer ersten Bohrung (8, 48, 61, 90) in benachbarte Bohrungen (10, 72, 91, 90, 91, 93) jeweils der benachbarte Bohrungen (8, 10, 48, 61, 72, 90, 91, 93) verbindende Kanal (36, 37; 42, 37; 42, 66, 67; 73, 75) am Boden der ersten Bohrung (8, 48, 61, 90) ansetzt und in die Deckfläche der benachbarten Bohrung (8, 10, 48, 61, 70, 90, 91, 93) mündet, wobei die Flüssigkeit in der ersten Bohrung (8, 48, 61, 90) jeweils unter dem Druck von Preßluft durch den Kanal (36, 37; 42, 37; 42, 66, 67; 73, 75) in die benachbarte Bohrung (8, 48, 61, 90) treibbar ist (Fig. 13, 16, 20, 23, 24).

5. Zentrifugationskammer nach Anspruch 4, dadurch gekennzeichnet, daß eine oder mehrere unterschiedliche Flüssigkeiten, z.B. Färbeflüssigkeit oder Fixierflüssigkeit in die erste Bohrung (8, 48, 61, 90) einleitbar sind, die nacheinanderfolgend über die, die benachbarten Bohrungen (8, 10, 48, 61, 72, 90, 91, 93) verbindenden Kanälen (36, 37; 42, 37, 42, 66, 67) in die in Serie hinter der ersten Bohrung (8, 48, 61, 90) geschalteten weiteren Bohrungen überleitbar sind (Fig. 21, 24).

6. Zentrifugationskammer nach Anspruch 5, dadurch gekennzeichnet, daß die genannte Einleitung und Überleitung von einer oder mehreren unterschiedlichen Flüssigkeiten in die nebeneinander angeordneten Bohrungen (90, 91, 93) während des Zentrifugationsvorganges erfolgt (Fig. 25).

7. Verwedung der Zentrifugationskammer nach einem der Ansprüche 1 oder 4 zur zytodiagnostischen Präparation von in einer Suspensionsprobe enthaltenen Epithelzellen, enthaltend

a) einen ersten Arbeitsgang, bei dem eine die zu untersuchende, Epithelzellen enthaltende Suspensionsprobe durch einen Zentrifugationsvorgang in der Zentrifugationskammer absedimentiert wird,

b) einem zweiten Arbeitsgang, bei dem das entstehende Zellsediment abgesaugt wird,

c) einen dritten Arbeitsgang, bei dem das abgesaugte Zellsediment auf ein Trennmedium überschichtet wird,

d) einen vierten Arbeitsgang, bei dem das auf das Trennmedium überschichtete Zellsediment einem Zentrifugationsvorgang unterworfen wird, wobei sich eine distale und proximale Fraktion bildet,

e) einen fünften Arbeitsgang, bei dem die distale und proximale Fraktion getrennt wird,

f) einen sechsten Arbeitsgang, bei dem die voneinander getrennte distale und proximale Fraktion in jeweils eine der genannten, parallel und im Abstand nebeneinander angeordneten und mit Probenflüssigkeit füllbaren Bohrungen (8, 9, 10; 9, 19, 18; 48, 10; 61, 72; 90, 91, 90, 91, 93) der Zentrifugationskammer eingebracht wird,

g) einen siebten Arbeitsgang, bei dem durch einen Zentrifugationsvorgang ein an der Zentrifugationskammer angeordneten Objektträger mit den in den genannten, parallel und im Abstand nebeneinander angeordneten und mit Probenflüssigkeit gefüllten Bohrungen (8, 9, 10; 9, 19, 18; 48, 10; 61, 10, 72; 90, 91, 90, 91, 93) anstehenden Epithelzellen der distalen und proximalen Fraktion an voneinander getrennten Flächen beschichtet wird,

h) einen achten Arbeitsgang, bei dem die auf den Objektträger aufsedimentierten Epithelzellen angefärbt werden, und

i) einen neuten Arbeitsgang, bei dem die präparierten, auf dem Objektträger angeordneten und angefärbten Epithelzellen einem optischen Untersuchungsgerät zugeführt werden,

dadurch gekennzeichnet, daß die obengenannten vierten bis sieben Arbeitsgänge d) bis g) während ein und demselben Zentrifugationsvorgang vorgenommen werden.

8. Verwendung nach Anspruch 7, dadurch gekennzeichnet, daß auch noch achte Arbeitsgang h) während des genannten einzigen Zentrifugationsvorgangs vorgenommen wird.

**Revendications**

1. Chambre de centrifugation pour la préparation, en vue d'un cytodiagnostic, de cellules épithéliales contenues en suspension dans un échantillon, constituée par une platine support (1) sur laquelle est fixé un porte-objet (2) sur le côté duquel est disposé de manière étanche une chambre d'essai (7, 30, 40, 50, 60, 100) qui présente de multiples perçages (8, 9, 10; 9, 19, 18; 48, 10; 61, 10, 72; 90, 91, 93) parallèles, séparés et disposés côte-à-côte, remplissables avec du liquide de préparation, dont les ouvertures frontales reposent toutes de manière étanche sur le porte-objet (2), caractérisée en ce que lesdits perçages (8, 9, 10; 9, 19, 18; 48, 10; 61, 10, 72; 90, 91, 93; 101) sont respectivement reliés entre eux par un conduit de liaison ayant la forme d'un canal (14, 15; 19, 20, 24; 36, 37; 42, 73, 74, 75, 76), ledit conduit de liaison étant constitué par un passage de débordement (14; 19, 20, 24) réglable en section, tandis que l'un de ces perçages (8, 9) comporte un premier perçage (8) de grand diamètre et un second perçage (9) de plus petit diamètre aligné avec le précédent (8), le passage de débordement (14; 19, 20, 24) prenant naissance dans le plan de raccordement entre ledit perçage de grand diamètre (8) et ledit perçage de petit diamètre (9) (fig. 1—3 et 5—9).

2. Chambre de centrifugation suivant la revendication 1, caractérisée en ce qu'une vis de freinage (15) est prévue pour modifier le passage de débordement (14) (fig. 1—3).

3. Chambre de centrifugation suivant la revendication 1, caractérisée en ce qu'il est prévu pour la modification de la section du passage de débordement (19, 20, 24) une pièce de freinage (22) déplaçable longitudinalement dans le sens axial des perçages (8, 9, 19), pièce dont la face frontale présente un canal de liaison radial (24) qui forme la liaison entre les deux passages de débordement (19, 20) (fig. 5—9).

4. Chambre de centrifugation pour la préparation, en vue d'un cytodiagnostic, de cellules épithéliales contenues en suspension dans un échantillon, constituée par une platine support (1) sur laquelle est fixé un porte-objet (2) sur le côté duquel est disposé de manière étanche une chambre d'essai (7, 30, 40, 50, 60, 100) qui présente de multiples perçages (8, 9, 10; 9, 19, 18; 48, 10; 61, 10, 72; 90, 91, 93) parallèles, séparés et disposés côte-à-côte, remplissables avec du liquide de préparation, dont les ouvertures frontales reposent toutes de manière étanche sur le porte-objet (2), caractérisée en ce que lesdits perçages (8, 9, 10; 9, 19, 18; 48, 10; 61, 10, 72; 20, 91, 93; 101) sont respectivement reliés entre eux par un conduit de liaison ayant la forme d'un canal (14, 15; 19, 20, 24; 36, 37; 42, 73, 74, 75, 76), ledit canal de liaison (36, 37; 42, 37; 42, 66, 67; 73, 75) destiné à conduire du liquide d'un premier perçage (8, 48, 61, 90) dans des perçages voisins (10, 72, 91, 90, 91, 93) prenant naissance au fond du premier perçage (8, 48, 61, 90) et débouchant dans le couvercle du perçage voisin correspondant (8, 10, 48, 61, 70, 90, 91, 93) tandis que ledit liquide qui se trouve dans le premier perçage (8, 61, 90) peut être éventuellement poussé sous l'effet de l'air comprimé à travers le canal correspondant (36, 37; 42, 37; 42, 66, 67; 73, 75) dans le perçage voisin (8, 48, 61, 90) (fig. 13, 16, 20, 23, 24).

5. Chambre de centrifugation suivant la revendication 4, caractérisé en ce qu'un ou plusieurs liquides différents, par exemple un liquide colorant ou un liquide de fixation, peuvent être introduits dans le premier perçage (8, 48, 61, 90), liquides qui peuvent être transmis en se suivant dans les autres perçages disposés en série derrière le premier perçage (8, 48, 61, 90) en traversant l'un après l'autre les canaux de liaison (36, 37; 42, 37; 62, 66, 67) reliant les perçages voisins (8, 10, 48, 61, 72, 90, 91, 93) (fig. 21, 24).

6. Chambre de centrifugation suivant la revendication 5, caractérisée en ce que lesdites introduction et transmission d'un ou plusieurs liquides différents dans les perçages disposés côte à côte (90, 91, 93) se produisent durant le processus de centrifugation (fig. 25).

7. Utilisation d'une chambre de centrifugation suivant l'une quelconque des revendications 1 à 4 pour la préparation, en vue d'un cytodiagnostic, de cellules épithéliales contenues en suspension dans un échantillon comportant:

a) un première phase opératoire au cours de laquelle un échantillon entourant en suspension les cellules épithéliales à examiner, est séparé par sédimentation au moyen d'un processus de centrifugation dans la chambre de centrifugation;

b) une deuxième phase opératoire au cours de laquelle le sédiment cellulaire obtenu est aspiré;

c) une troisième phase opératoire au cours de laquelle le sédiment cellulaire aspiré est versé sur un milieu de séparation;

d) une quatrième phase opératoire au cours de laquelle le sédiment cellulaire versé sur le milieu de séparation est soumis à un processus de centrifugation au cours duquel se forment une fraction distale et une fraction proximale;

e) une cinquième phase opératoire au cours de laquelle la fraction distale est séparée de la fraction proximale;

f) une sixième phase opératoire au cours de laquelle les fractions séparées distale et proximale sont respectivement introduites chacune dans l'un desdits perçages (8, 9, 10; 9, 19, 18; 48, 10; 61, 10, 72; 90, 91, 90, 91, 93) séparés, disposés parallèlement et côte-à-côte, et remplissables avec du liquide d'essai, de la chambre de centrifugation,

g) une septième phase opératoire au cours de laquelle, par un processus de centrifugation, un porte-objet disposé dans la chambre de centrifugation est recouvert sur des parties de surface distinctes et séparées, respectivement par les cellules épithéliales provenant des fractions distale et proximale contenues dans lesdits perçages (8, 9, 10; 9, 19, 18; 48, 10; 61, 72; 90, 91, 90, 91, 93) parallèles, séparés, disposés côte-à-côte et remplis de liquide d'essai;

h) une huitième phase opératoire au cours de laquelle les cellules épithéliales disposées par sédimentation sur le porte-objet sont colorées, et

i) une neuvième phase opératoire au cours de laquelle les cellules épithéliales préparées, disposées sur le porte-objet et colorées sont amenées sous un appareil optique d'examen, caractérisée en ce que les quatrième à septième phases opératoires ci-dessus désignées, soit de d) à g), sont réalisées durant un seul et unique processus de centrifugation.

8. Utilisation suivant la revendication 7, caractérisée en ce que la huitième phase opératoire, soit h) est également réalisée durant ledit seul et unique processus de centrifugation.

**Claims**

1. Centrifugation chamber for cytodiagnostic preparation of epithelial cellš contained in a suspension sample, consisting of a carrier plate (1) and, attached thereto, a specimen slide (2), sealed onto the side of which sits a sample chamber (7, 30, 40, 50, 60, 100), which has several bores (8, 9, 10; 9, 19, 18; 48, 10; 61, 10, 72; 90, 91, 93) which are arranged parallel, side by side and spaced from one another and can be filled with sample liquid and of which the openings on the front face each sit tightly on the specimen slide (2), characterised in that a flow connection in the form of a channel (14, 15; 19, 20, 24; 36, 37; 42; 73, 74, 75, 76) is in each case arranged between these bores (8, 9, 10; 9, 19, 18; 48, 10; 61, 10, 72; 90, 91, 93; 101), this flow connection consisting of an overflow bore (14; 19, 20, 24) of variable cross-section, one bore (8, 9) being divided into a bore (8) of larger diameter and a bore (9) of smaller diameter which is aligned with this (8), and the overflow bore (14; 19, 20, 24) issuing from the transition from the larger (8) to the smaller bore (9) (Figs. 1—3 and 5—9).

2. Centrifugation chamber according to claim 1, characterised in that a clamping screw (15) is provided for changing the cross-section of the overflow bore (14) (Fig. 1—3).

3. Centrifugation chamber according to claim 1, characterised in that a clamping piece (22) which can be shifted axially in the longitudinal direction of the bores (8, 9, 19) and on the front face of which is a radial connecting channel (24) which forms the connection between the two overflow bores (19, 20), is provided for changing the cross-section of the overflow bore (19, 20, 24) (Fig. 5—9).

4. Centrifugation chamber for cytodiagnostic preparation of epithelial cells contained in a suspension sample, consisting of a carrier plate (1) and, attached thereto a specimen slide (2), sealed onto the side of which sits a sample chamber (7, 30, 40, 50, 60, 100), which has several bores (8, 9, 10; 9, 19, 18; 48, 10; 61, 10, 72; 90, 91, 93) which are arranged parallel, side by side and at a distance from one another and can be filled with sample liquid and of which the openings of the front face each sit tightly on the specimen slide (2), characterised in that a flow connection in the form of a channel (14, 15; 19, 20, 24; 36, 37; 42; 73, 74, 75, 76) is in each case arranged between these bores (8, 9, 10; 9, 19, 18; 48, 10; 61, 10, 72; 90, 91, 93; 101), wherein, for transfer of liquid from a first bore (8, 48, 61, 90) into adjacent bores (10, 72, 91, 90, 91, 93) in each case the channel (36, 37; 42, 37; 42, 66, 67; 73, 75) connecting the adjacent bores (8, 10, 48, 61, 72, 90, 91, 93) joins at the base of the first bore (8, 48, 61, 90) and enters in the top of the adjacent bore (8, 10, 48, 61, 70, 90, 91, 93), it being possible for the liquid in the first bore (8, 48, 61, 90) in each case to be driven under the pressure of compressed air through the channel (36, 37; 42, 37; 42, 66, 67; 73, 75) into the adjacent bore (8, 48, 61, 90) (Fig. 13, 16, 20, 23, 24).

5. Centrifugation chamber according to claim 4, characterised in that one or more different liquids, e.g. staining liquid or fixing liquid, can be introduced into the first bore (8, 48, 61, 90) and can be transferred in succession via the channels (36, 37; 42, 37; 42, 66, 67) connecting the adjacent bores (8, 10, 48, 61, 72, 90, 91, 93) into the subsequent bores connected in series downstream of the first bore (8, 48, 61, 90) (Fig. 21, 24).

6. Centrifugation chamber according to claim 5, characterised in that the introduction and transfer mentioned for one or more different liquids in the bores (90, 91, 93) arranged in succession take place during the centrifugation operation (Fig. 25).

7. Use of the centrifugation chamber according either of claims 1 or 4 for cytodiagnostic preparation of epithelial cells contained in a suspension sample, comprising

a) a first operation in which a suspension sample containing the epithelial cells under investigation is sedimented by a centrifugation operation in the centrifugation chamber,

b) a second operation in which the cell sediment formed is filtered off with suction,

c) a third operation in which the cell sediment filtered off with suction is introduced in a layer over a separating medium,

d) a fourth operation in which the cell sediment introduced in a layer over the separating medium is subjected to a centrifugation operation, a distal and a proximal fraction being formed,

e) a fifth operation in which the distal and proximal fractions are separated,

f) a sixth operation in which the separated distal and proximal fractions are introduced into in each case one of the centrifugation chamber bores (8, 9, 10; 9, 19, 18; 48, 10; 61, 10, 72; 90, 91 90, 91, 93) mentioned which are arranged parallel, side by side and spaced from one another and can be filled with sample liquid,

g) a seventh operation in which, by a centrifugation operation, a specimen slide arranged on the centrifugation chamber is coated on separate areas with the epithelial cells of the distal and proximal fraction which are waiting in the bores (8, 9, 10; 9, 19, 18; 48, 10; 61, 10, 72; 90, 91, 90, 91, 93) mentioned, which are arranged in parallel, side by side and spaced from one another and are filled with sample liquid,

h) an eighth operation in which the epithelial cells sedimented onto the specimen slide are stained, and

i) a ninth operation in which the prepared epithelial cells arranged and stained on the specimen slide are passed to an optical examination unit,

characterised in that the abovementioned fourth to seventh operations d) to g) are carried out during one and the same centrifugation operation.

8. Use according to claim 7, characterised in that the eighth operation h) is also carried out during the single centrifugation operation mentioned.

0 105 194

FIG 1

FIG 2

FIG 3

Probe
Plasma steril

12 prox Frakt.
Fraktionierung
16 (dist.Frakt.)

FIG 4

1

0 105 194

FIG 5

FIG 6

FIG 7

FIG 8

FIG 9

2

XI

44

28    27    25

29    26

FIG 10

27

25

28

29    26

FIG 12

28  44  27  26

25

29

29

28    27

FIG 11

0 105 194

FIG 13

FIG 14

FIG 15

4

FIG 16

FIG 17

FIG 18

FIG 19

FIG 20

FIG 21

FIG 22

FIG 23

FIG 24

FIG 25